# EUROPEAN PATENT APPLICATION

(11) **EP 3 058 926 A1**
(43) Date of publication of application: **24.08.2016**
(21) Application number: 15155646.1
(22) Date of filing: 18.02.2015
(51) Int. Cl.: A61H 3/06, A61F 9/08, G09B 21/00

(54) **Method of transforming visual data into acoustic signals and aid device for visually impaired or blind persons**

(71) Applicant: Technische Universität München, 80333 München (DE)
(72) Inventor: Conradt, Jörg, 80637 München (DE); Gadheri, Viviane, 81669 München (DE); Mulas, Marcello, 80807 München (DE); Santos Pereira, Vinicius Felisberto, 35. 164-257 Ipatinga MG (BR); Weikersdorfer, David, 94301 Palo Alto, California (US)
(74) Representative: Hoefer & Partner Patentanwälte mbB

(57) **Abstract**

The present invention concerns a method of transforming raw visual data, or 3D (depth) visual data, or an interpreted visually perceived 3D scene into acoustic signals for aiding visually impaired or blind persons, comprising the steps of capturing visual environment data by at least one vision sensor unit, wherein the at least one vision sensor unit is formed as an event-based vision sensor, transforming the captured visual environment data to acoustic signals, and outputting the acoustic signals to the visually impaired or blind person by at least an audio output unit. Another aspect of the present invention concerns an aid device that comprises means to carry out the method of the present invention.

## Description

The present invention concerns a method of transforming visual data into acoustic signals and an aid device for visually impaired or blind persons.

Several efforts have been made in the past for providing mobility aids that aimed to help visually impaired or blind people to overcome difficulties and challenges that arise in their daily life. The most commonly used mobility aid is the inexpensive traditional long (white) cane which has been in use for decades to assist visually impaired people to attain spatial information unperceivable through their other sensory modalities. The limitations of the long cane include short range, limited resolution, and the inability to detect obstacles higher than hip-level. Guide-dogs do not have these limitations, but they are very expensive due to extensive training they require to safely guide a visually impaired person. Furthermore, guide-dogs need a lot more overhead in terms of maintenance and resources.

Electronic travel aids (ETAs) aim to address the limitations of existing mobility aids to improve safe navigation for visually impaired people via sensory substitution. ETAs capture information from the spatial environment and convey this information through another sensory modality, such as touch or sound. Existing ETAs are currently not widely adopted, because of reasons such as low battery lifetime, low reaction time and limited portability of the devices, unintuitive representations, and long training time.

Most of the currently commercially available ETAs assist visually impaired people to navigate and avoid obstacles by translating visual spatial information to touch (vibration patterns). These devices are often integrated into the traditional collapsible white cane or are standalone used in combination with the white cane.

Despite some improvement in independent navigation for the visually impaired user, these devices are not widely adapted. One of the main problems with ETAs, which translate vision-to-touch, is the low resolution of touch (low density of touch receptors on the body), as compared to the resolution of vision and hearing. Therefore, the amount of information that can be conveyed by such devices is limited. Other disadvantages are long training times, bulk equipment and high devices cost for only a limited gain in mobility.

ETAs currently under development that translate visual data to sounds have several disadvantages. First of all, image and video processing requires a significant amount of computing resources, as the current devices translate visual input from a regular camera into audio patterns.

Therefore, vision-to-sound ETAs which use regular cameras include processing hardware, which consumes large amounts of power and consequently, the devices suffer from low battery life-time. Furthermore, devices, which translate images into a panning sound pattern from left to right, require training times of several months for a motivated user. The audio information provided to the user is very complex. Hearing plays a vital role in the daily lives of visually impaired people. Such complex audio inputs can easily lead to sensory overload and distract the user.

It is therefore an objective underlying the present invention to provide an easily applicable and simple method of transforming visual data to acoustic signals for a visually impaired or blind person that can generate a simplified spatial representation which does not lead to an overstimulation of the person. It is another objective underlying the present invention to provide an improved aid device in terms of its cost, size and weight that can carry out the method of the invention in an easy and user-friendly way.

The objective is achieved by a method for transforming visual data into acoustic signals according to claim 1. The inventive method comprises the steps of capturing visual environment data by at least one vision sensor unit, wherein the at least one vision sensor unit is formed as an event-based vision sensor, transforming the captured visual environment data to acoustic signals, and outputting the acoustic signals to the visually impaired or blind person by at least an audio output unit.

In contrast to conventional image sensors that produce massive amounts of redundant data and are limited in temporal resolution by the frame rate, event-based sensors record, similar to the human eye, only changes in a given scene. These changes are called events. Thus, the inventive method produces a significantly lower amount of acoustic signals, which on one hand protects the visually impaired or blind people from being acoustically overstimulated and at the same enables them to get used to the inventive aid device faster. Moreover, as the event-based sensors are not based on capturing frames, event-based signals can be processed more efficiently because there is less data to be processed. This allows the use of energy-efficient hardware that can process event-based data in real time.

The sub-claims contain advantageous embodiments of the present invention.

According to an embodiment of the present invention, the method of transforming visual data to acoustic signals comprises the steps of stereoscopically capturing visual environment data by at least two vision sensor units, wherein the at least two vision sensor units are formed as event-based vision sensors, extracting 3D visual information from the captured visual environment data, transforming the 3D visual information into a 3D stereo sound landscape, and outputting the 3D stereo sound landscape to the visually impaired or blind person by at least a stereo output unit.

The term "3D visual information" refers to a visual representation that also includes the depth of a 3D image, which can be calculated from stereoscopically taken images of a scene. Thus, the transformation of visual data to acoustic signals is based on information about the distance of an object from the visually impaired or blind person. The person can perceive basic information about 3D space in a natural way.

The term "3D stereo sound landscape" refers to what is actually heard by the visually impaired or blind person and is comprised of a plurality of sounds that represent "3D visual information" (as defined above) of the environment in 3D.

A particular embodiment of such sounds can be a click. By the term "click" a short sound pulse of a specific frequency is meant, which has duration of approximately a few milliseconds and is spaced from another pulse by a few milliseconds. The amplitude among other aspects of the click such as duration or frequency varies depending on the 3D spatial location of the corresponding event, i.e. from the 3D visual information containing information about the depth of an image.

The 3D stereo sound landscape can comprise also other sounds than clicks. These sounds can be preferably recorded and reproduced in the 3D sound landscape after they have been assigned to visual data based on predefined criteria. It also possible to generate new sounds using different pulses or waves with different frequencies.

By transforming visual data into a 3D stereo sound landscape, no panning is used, which results in a more intuitive perception of the space through the acoustic signals for the visually impaired or blind person.

By using two event-based sensors, a more precise but still simple-to-understand representation of spatial structure can be achieved.

According to an embodiment of the present invention, only a part of the extracted 3D visual information is preferably selected to be transformed into the 3D stereo sound landscape. In other words, not all the events detected by the event-based sensors are processed. Thus, events that may arise from background activity or redundant sources, and would otherwise produce unnecessarily large amounts of data (but still less, compared to the data produced using conventional sensors) and possibly confuse the visually impaired or blind person are discarded. Reducing content of the 3D environment can be based on some metric, e.g. "distance" (only keep nearby objects), or "velocity" (only keep fast moving objects) or "size" (only big objects) etc. This way 3D world can be partially ignored and thus, only the important part thereof can be transformed into the 3D stereo sound landscape. This leads to the generation of acoustic signals that represent the 3D scene of reduced complexity so as not to overload the visually impaired or blind person. However, it is also possible to process all events in order to transform them into acoustic signals. With that, a very rich 3D spatial representation of the local environment can be achieved, taking as many events as possible to create a reliable and complete representation of the 3D environment.

According to another embodiment of the present invention, an amount of the captured visual environment data or the extracted 3D visual information to be transformed is selected. The amount may refer to a continuous range of "complexity" of the sound representation. Thus, the visually impaired or blind person can adjust his/her preferred level of acoustic signaling. This could be e.g. done by a slider or fader selecting the amount of information being transformed from visual data to acoustic signals. So the blind person can adjust his or her preferred level / amount of acoustic signaling.

If e.g. blind people operate in well-known and safe environments such as inside their home, they can switch to a "detailed" mode and thereby gain a lot of detailed acoustic information.

If - in contrast - they operate in unknown and potentially dangerous environments such as a street with bikes and/or cars), they might want to reduce the granularity and only get important information conveyed.

As a third aspect users might be tired and such feel more comfortable using the device with only a limited amount of acoustic information.

According to another embodiment of the present invention, a first mode of operation, in which the output acoustic signals are minimized, or a second mode of operation, in which the output acoustic signals are maximized, are carried out. In cases, where e.g. a large amount of acoustic signals is needed, the inventive method is carried out in the second mode. On the other hand, when the situation is such that only a limited number of acoustic signals is required or wanted, the inventive method is carried out in the first mode. The maximization or minimization of acoustic signals applies to the case where all the extracted 3D information, namely all the events, are chosen as well as to the case where only a part of the extracted 3D information is selected.

If environmental sounds are important, as is the case when a person is walking on a street, the first mode could be selected. In such a case, only acoustic signals in the form of warnings are produced. On the other hand, if environmental sounds are not considered to be of importance and if the inventive aid device is not to be used for navigation purposes, the second mode is preferably used.

If navigation is desired, a third mode of operation is preferably provided. In the third mode the amount of transformed information is continuously adjustable between the first mode and the second mode, i.e. between a minimized amount and a maximized amount of acoustic signals.

According to another embodiment of the present invention, the first mode is preferably carried out at a low resolution of the extracted 3D visual information and/or a low sound output rate and/or a low throughput of acoustic signals. The second mode is preferably carried out at a high resolution of the extracted 3D visual information and/or a high sound output rate and/or a high throughput of acoustic signals. The resolution refers to the spatial resolution at which the 3D visual information is in the end transformed to acoustic signals. The higher the chosen resolution is, the more points of the images captured by the vision sensors are used for the transformation of the 3D visual information to acoustic signals. The full throughput of acoustic signals can be achieved by using all the available events, based on which the acoustic signals are produced.

According to another embodiment, an amount of the extracted 3D visual information to be transformed can be preferably selected either manually by the visually impaired or blind person through a user interface or automatically upon detection of the environment by the visual sensor units and/or at least one audio sensor unit. This enables the person to select the preferred amount of visual information depending on the respective situation or environment. The amount of visual information could be also automatically chosen e.g. when a certain environment, in which the person had been before, is detected. Also, other criteria, e.g. if the extracted 3D visual information is below a predefined threshold, can be preferably applied in order to choose between the available modes.

According to another embodiment of the present invention, the processing steps of velocity extraction and/or shape detection from and/or clustering of the extracted 3D visual information and the like are preferably carried out. These steps can all be applied to the same event or group of events. It is, however, also possible to separately apply different steps to different events. For example, in case of a moving object in the captured visual scene, velocity data could be extracted from the extracted 3D visual information corresponding to the group of events. On the other hand, for helping a person identify an object, the step of shape detection is preferably applied.

To improve the quality of the transformation of visual data into acoustic signals, it is advantageous in the present invention to remove noise from the captured visual environment data and/or the extracted 3D visual information. This ensures that any artifacts present in the images taken by the vision sensor units or the reconstructed 3D image are eliminated or reduced. Thus, only the significant visual data get to be transformed into acoustic signals.

Advantageously, the 3D stereo sound landscape is encoded by using a head-related transfer function, and/or with added reverberations, and/or by a binaural room response. A head-related transfer function relates a sound in space to a person's ear canal and is individualized based on bodily features of that person e.g. ear size or head size. Thereby, the transformation of the visual data into acoustic signals is personalized and thus, the person may perceive the transformed acoustic signals, the way he or she perceives real sounds in 3D space. The head-related transfer function does not account for environmental features. The influence of the environment on how a sound is perceived can be taken into account by encoding the 3D stereo sound landscape using reverberations or reflections. In order to consider the impact of room acoustics on sound perception in the transformation of visual data into acoustic signals, a binaural room response or also known as binaural room impulse response can also be preferably used. Thus, the impulse response that a real sound would have from a source position to the listener's left and right ears is modeled in the transformation of the visual data into acoustic signals.

According to a further embodiment of the present invention, characteristic sound representations that represent particular objects corresponding to visual environment data are incorporated into the 3D stereo sound landscape. Each time that a particular object is identified by the vision sensor units or within the 3D visual representation, the characteristic sound representation for that object that is already stored in a database and/or even an acoustic message can be output to the visually impaired person. What the characteristic representation sound means in terms of the presence of an object in the environment would be known to the visually impaired person beforehand. A characteristic representation sound is not necessarily the sound that the object actually produces but typically a predetermined elementary sound associated with that object. This enables the visually impaired or blind person to quickly identify that particular object.

The steps of capturing the visual environment data and transforming the captured visual environment data into acoustic signals are advantageously carried out in-real time. Real time means that the time an event happens and the time the user hears a sound is perceived as instantaneous. In other words, the time difference is preferably at most on the order of a few hundred milliseconds. The term "transformation of the captured visual environment data" refers to all the steps that are carried out after the visual environment data is captured and not only to the actual transformation into the 3D stereo sound landscape.

The present invention further concerns an aid device, more particularly a travel electronic aid in the form of eye glasses, for visually impaired or blind persons, which comprises means for carrying out the inventive method. The aid device comprises preferably a processing unit that is configured to carry out the method of the present invention. It is understood that more processing units can be provided in the aid device. Each of the processing units may carry out all the steps of the inventive method. I.e. each step can be carried out in a parallel way from more than one processing units. Alternatively, one processing unit may carry only one or several step of the inventive method.

The aid device provides intuitive audio output that does not require long training times. As only the necessary data is processed, the aid device can be made compact and light-weight by using a smaller processing unit.

It is preferable that the at least one vision sensor unit formed as event-based vision sensor is dynamic vision sensor. Dynamic vision sensors are reliable, operate under vastly varying light conditions, and achieve a drastic reduction in power, data storage and computational requirements.

According to another embodiment, the inventive aid device further comprises an audio input unit, which is configured to be used for voice control of the device. Thus, the aid device can be controlled without using hands. This allows the visually impaired or blind persons to use their hands for balance or for holding a white cane as an additional aid device.

Preferably, the aid device further comprises an audio output unit that has at least an ear headphone and/or at least a bone-conducting headphone. An ear headphone is cost efficient, whereas a bone-conducting headphone provides an ear-free use of the aid device. Thus, the person can perceive all the natural sounds in the environment.

According to another embodiment, the inventive aid device further comprises a housing, wherein all components of the device are integrated in the housing. This results in a compact design of the aid device, that it is easily carried and used. Furthermore, such a device is safe for the user, as there are no components or cables connecting the different components to each other outside of the housing. This increases also the lifetime of the device, as no component is susceptible to environmental conditions.

In another embodiment of the present invention, three or more vision sensor units are provided and arranged in such a way that at least a set of three vision sensor units define a plane. In other words, the three vision sensor units do not lie on the same line, namely they are not arranged collinearly. This leads to more accurate results with regard to the extracted 3D visual information. With more than three vision sensor units a higher spatial precision and/or a larger field-of-view can be achieved.

Further details, advantages and characteristics of the present invention will be explained with respect to the following description of the embodiments in light of the figures. The figures show:
- Fig. 1: a schematic, simplified perspective view of an aid device in the form of eye glasses according to a first embodiment,
- Fig. 2: a schematic, simplified front view of the aid device of Fig. 1,
- Fig. 3: a schematic, simplified side view of the aid device of Fig. 1,
- Fig. 4: a schematic, simplified perspective view of an aid device in the form of eye glasses according to a second embodiment,
- Fig. 5: a schematic, simplified perspective view of an aid device in the form of eye glasses according to a third embodiment,
- Fig. 6: a schematic, simplified perspective view of an aid device in the form of eye glasses according to a fourth embodiment,
- Fig. 7: a schematic, simplified perspective view of an aid device in the form of eye glasses according to a fifth embodiment,
- Fig. 8: a schematic, simplified perspective view of an aid device in the form of eye glasses according to a sixth embodiment,
- Fig. 9: a schematic, simplified perspective view of an aid device in the form of eye glasses according to a seventh embodiment,
- Fig. 10: a schematic, simplified perspective view of an aid device in the form of eye glasses according to an eighth embodiment,
- Fig. 11: a flow diagram showing an embodiment of the method of the present invention, and
- Fig. 12: a decision flow diagram showing different modes for carrying out the method of the present invention.

The present invention is described with reference to the following figures. Herein all essential elements and components of the inventive aid device and method are shown. All other elements and components have been omitted to increase the understanding of the present invention. In the figures the same reference numbers denominate the same elements/components.

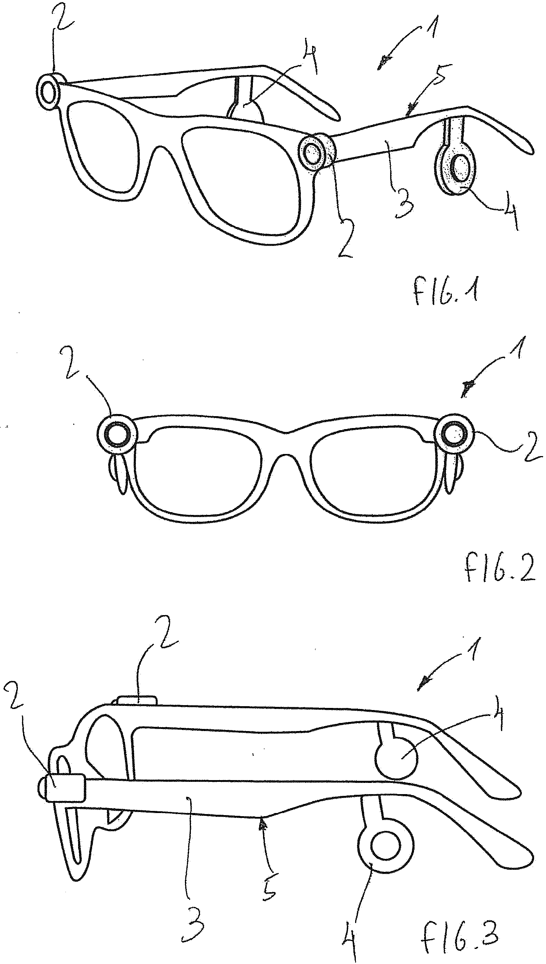

Figures 1, 2 and 3 show a schematic, simplified perspective view of an aid device 1 for a visually impaired or blind person according to a first embodiment of the present invention. The aid device 1, which is formed as eye glasses, comprises two vision sensor units 2, a processing unit 3, and two stereo output units 4.

In particular, the vision sensor units 2 are configured to stereoscopically capture visual environment data and are formed of event-based sensors, more particularly dynamic vision sensors. This specific type of sensors captures only changes that occur in the environment and thus produces a significantly lower amount of data to be further processed. Thereby, event-based sensors can be used in more real-world situations. Furthermore, the use of event-based sensors can lead to a great decrease of costs and power consumption.

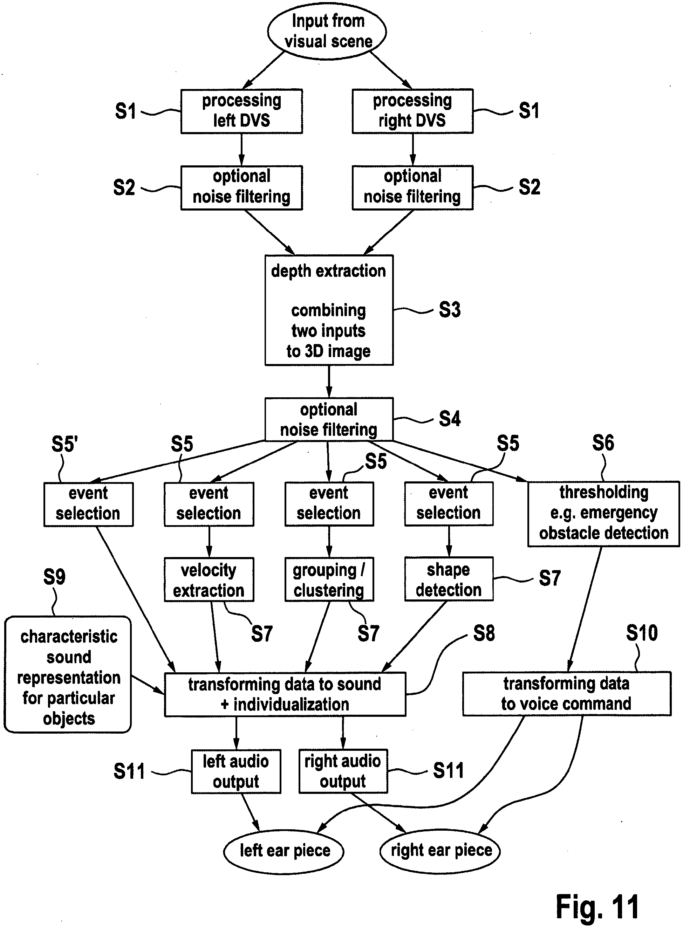

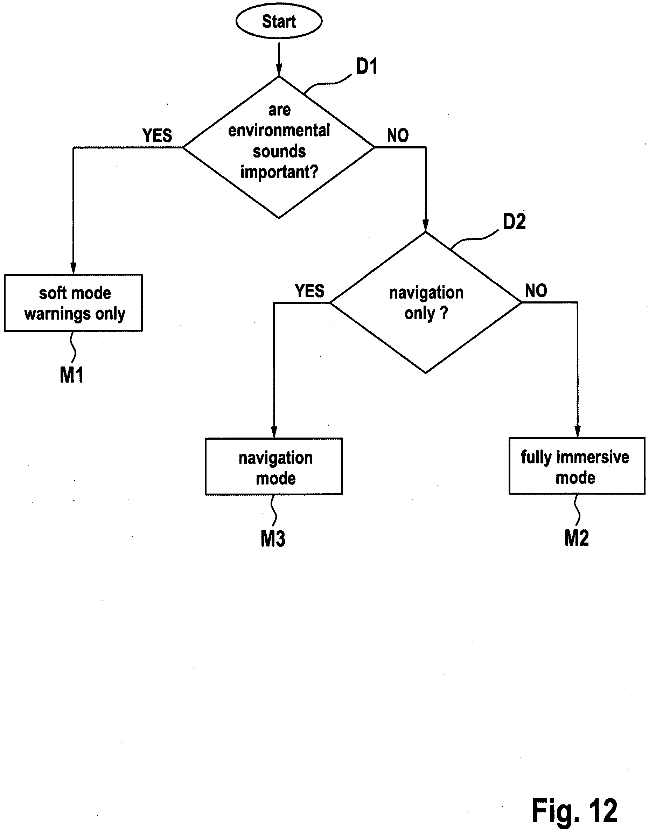

The processing unit 3 is configured to transform the visual environment data captured from the two vision sensor units 2 into acoustic signals, which are then output to the visually impaired or blind person via the stereo output units 4. In a first step of the transformation, 3D visual information is extracted from the captured visual environment data. The 3D extracted visual data is then transformed into a 3D stereo sound landscape. The method of transforming visual data to acoustic signals will be described later in detail on the basis of figures 11 and 12.

The processing unit 3 is integrated in a housing 5 in the form of a frame of the eye glasses, whereas the stereo output units 4, which are formed as bone-conducting headphones, are attached to the frame from the outside. The vision sensor units 2 are arranged on a front side of the frame. The position of the vision sensor units 2 can vary depending on their view range as well as the anatomic characteristics of the user of the aid device.

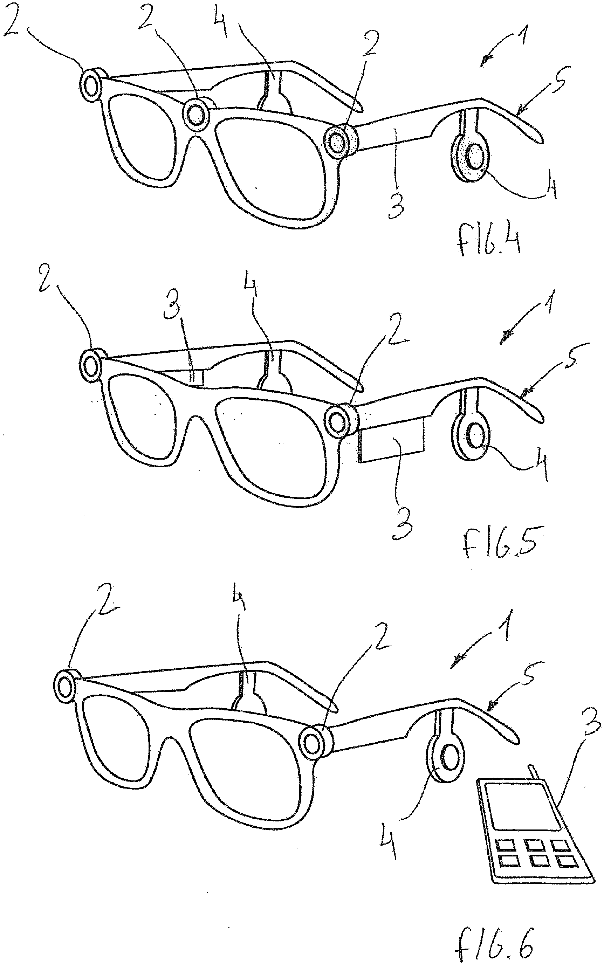

Figure 4 shows a schematic, simplified perspective view of an aid device 1 in the form of eye glasses according to a second embodiment of the present invention. The eye glasses of figure 4 differ from the eye glasses of figures 1 to 3 in that they comprise three vision sensor units 2. As can be seen in figure 4, the three vision sensor units 2 are arranged on the same line. Alternatively, the three vision sensor units 2 can be arranged in non-collinear way. This leads to better 3D extracted visual data and consequently to more accurate acoustic signals, which help the visually impaired or blind person using the device perceive the environment in a more realistic way. Also, it is possible that the aid device 1 comprises more than two vision sensor units 2.

In a third embodiment according to the present invention, which is shown in figure 5, two processing units 3 are provided in the aid device 1 and are both attached to the frame or housing 5 from the outside. This enables an easier access to the processing units 3, if the processing units 3 need to be checked, repaired or replaced.

The processing unit 3 of the aid device 1 according to a fourth embodiment of the present invention (figure 6) is a separate, external element providing remote processing of the captured visual environment data and remote transmission of the processed data with the aid device 1. Furthermore, the processing unit 3 can be used as a remote control or a user interface, through which a user of the aid device 1 can easily control the operation of the aid device 1.

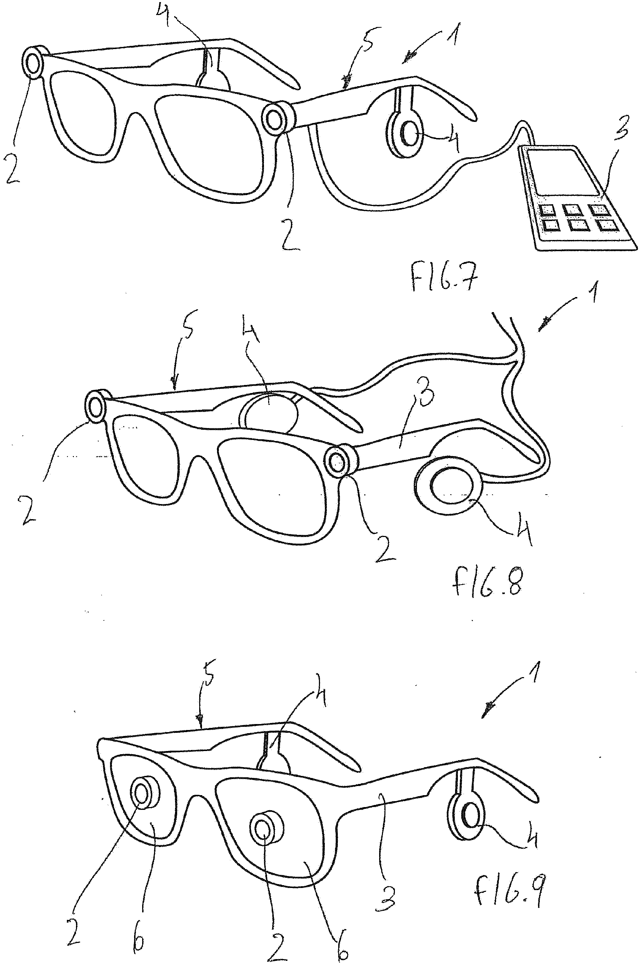

As can be seen in figure 7, the separate processing unit 3 can also be connected via a wire to the aid device 1 according to a fifth embodiment of the present invention. Again, the processing unit 3 may act also as a remote control for the aid device 1. The wired connection between the processing unit 3 and the aid device 1 may be advantageous for people that are afraid of losing the processing unit 3 or in cases where a wireless transmission is not preferred e.g. for health reasons.

In a sixth embodiment according to the present invention (figure 8), the stereo output units 4 in the form of bone-conducting headphones are designed as external units. This facilitates an easy personalized placement of the stereo output units 4 on the head of the person using the aid device 1.

An eighth embodiment of figure 9 differs from the preceding embodiments in that the vision sensor units 2 are arranged on the lenses 6 of the eye glasses.

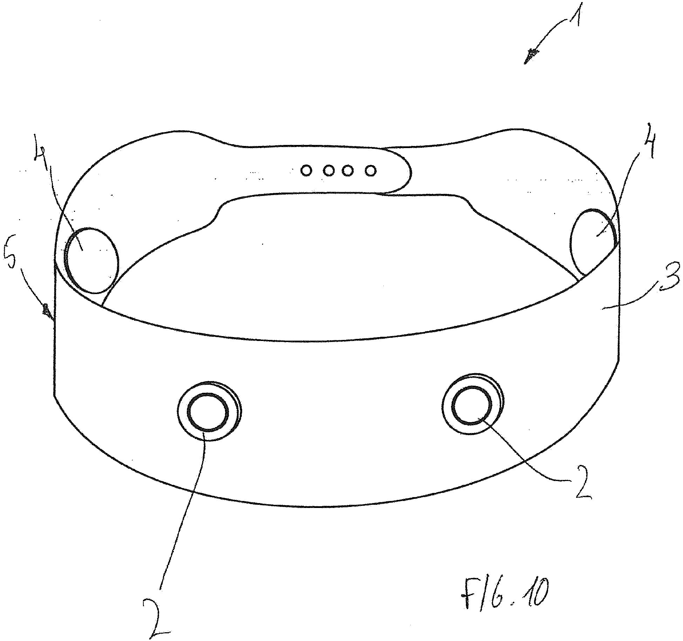

The aid device 1 can be also in a form of a headband, as shown in figure 10. The two vision sensor units 2 are arranged on a front side of the headband, when the headband is placed on the head of a person, whereas the stereo output units 4 in the form of bone-conducting headphones are placed on an inner side of the headband to be in contact with the person's head. The realization of the aid device 1 as a headband enables an easy adjustment of the aid device 1 to different people. Instead of only two sensor units 2, it is also possible to provide the headband with more sensor units 2 that are distributed all around the periphery of the headband. Through such an arrangement an up to 360° vision field can be achieved.

As already briefly mentioned, the aid devices 1 of figures 1 to 10 are configured to capture visual environment data and transform the captured visual environment data into acoustic signals for helping visually impaired or blind people. An embodiment of the inventive method, according to which the transformation from visual data into acoustic signals is carried out, will now be described in detail on the basis of figures 11 and 12.

More specifically, figure 11 shows a flow diagram showing the different steps of the method of the present invention. In figure 12, a decision flow diagram showing different modes of operation according to the method of the present invention is presented.

In step S1 of the inventive method, each of the vision sensor units 2 formed as left and right dynamic vision sensors captures a visual scene of the environment or visual environment data. In particular, the vision sensor units 2 respond to temporal contrast rather than absolute illumination in the captured visual scene. Thus, the output of the vision sensor units 2 at a given time consists of two images that comprise pixels that signal reflectance changes of the visual scene in real time. The images containing the scene changes or also known as events are herein referred to as captured visual environment data.

The captured visual environment data is then filtered for noise removal in step S2. Though this step is optional, it may be considered necessary, if the captured visual environment data contains a lot of artifacts. By filtering out the noise, the accuracy of the captured visual environment data is increased.

In step S3 of the method, the images derived from the two vision sensor units 2 are combined in order to reconstruct a 3D image. In other words, depth values of the captured visual scene are calculated on the basis of the captured visual environment data from the left and the right vision sensor units 2. The depth values actually refer to the distances of the captured objects that are present in the visual scene from a reference point defined by taking into account the position of the vision sensor units 2 in space. The output of step S3 is herein referred to as extracted 3D visual information and contains depth values of all captured events. Subsequently, a further noise removal step is carried out (step S4), so that artifacts produced by the reconstruction of the 3D image can be reduced or even eliminated. This step is, however, optional.

In step S5, only a part of the filtered captured events is selected for further processing. It is though also possible to consider all events in step S5 for producing the 3D stereo sound landscape. In step S5', the selected events (corresponding to either only a part of or the total number of events) are directly used to generate acoustic signals, whereas the selected events in step S5 are first interpreted in step S7 and then transformed into acoustic signals, as explained next.

In step S7, velocity extraction, grouping and shape detection are carried out as examples for processing of the selected events of step S6. However, any number of processing streams is possible, including a processing stream that simply forwards all events (no processing). Each such processing stream can access all events or decide to rely on a subset of events only. By choosing a subset, a sensory overstimulation of the visually impaired or blind person can be avoided.

The outputs of the processes carried out in step S7 are then transformed into a 3D stereo sound landscape. The 3D stereo sound landscape is encoded by using e.g. a head-related transfer function, so that the 3D stereo sound landscape is individualized for the user of the aid device 1 according to his or her bodily features. If the visually impaired or blind person using the aid device 1 is e.g. in a closed room, the 3D stereo sound landscape can be encoded by using reverberations in addition to the head-related transfer function, or a binaural room response. With that, the influence of the room on the perception of the direction from which a sound comes is modeled. Thus, the person can have a better understanding of the location of an object in space through the modified acoustic signal.

In step S9 characteristic sound representations that represent particular objects are introduced into the 3D stereo sound landscape. Objects, especially machines or vehicles may produce a sound that is characteristic for this object only and that can be used to identify that object. If an object or, to be more precise, a change in such an object is identified by the vision sensor units 2 or within the 3D visual representation, a characteristic sound representation for the object can be introduced into the 3D stereo sound landscape. A characteristic sound representation does not need to be the actual sound that the object produces. The characteristic sound representations for different objects e.g. vehicles can be already stored in a database or memory of the processing unit 3.

Finally, the individualized 3D stereo sound landscape is reproduced in the stereo output units 4 in Step S11 and output to the left and right ear of the visually impaired or blind person. Thus, the person is able to indirectly perceive visual environment structure through acoustic signals.

In step S6, a thresholding process is applied to the filtered extracted 3D visual information (steps S3 and S4), where only the events or the filtered extracted 3D visual information which exceed a predetermined and adjustable threshold value, are considered. For example, this is useful in order to facilitate e.g. an emergency obstacle detection and thus, protect the visually impaired or blind person from hitting an object. As long as an emergency situation is detected based on the captured visual environment data, the processing unit 3 automatically carries out step S6. After the thresholding process in step S6 the remaining events are transformed to voice commands (S10) and output to the ears of the person via the output stereo units 4. The emergency obstacle detection process shall continuously run in parallel to standard operation of the generation of the 3D stereo sound landscape; and whenever an emergency is detected an additional signal (e.g. voice command) shall be issued.

The aid device 1 of the present invention can carry out the described method in several modes. In figure 12, three modes M1, M2 and M3 are presented as examples. A different implementation can feature continuously adjustable amount of information in the auditory representation.

If environmental sounds are considered to be important (question D1) e.g. sounds of vehicles, when the visually impaired or blind person is walking in an outside environment, a first mode M1 of operation is carried out, in which the produced audio signals are minimized and only acoustic warnings are output to the person.

Should environmental sounds be regarded as less important, it is judged whether the aid device 1 should be used only for navigation purposes (question D2). If the answer to question D2 is negative, a second mode of operation M2 (fully immersive mode) is chosen, in which the acoustic signals are maximized. If the answer to question D2 is positive, the aid device 1 is operated in a third mode M3 (navigation mode). In the third mode the amount of transformed information can be adjusted in a continuous manner between the first mode and the second mode. The user of the aid device 1 can namely choose any amount of output acoustic signals between a minimum and a maximum according to his needs.

The answer to questions D1 and D2, i.e. whether the first, the second or the third mode of operation will be chosen, can be automatically decided by the aid device 1 based on the captured visual environment data. This means that the different modes can dynamically be altered according to the captured visual environment data. Also, it is possible to control the modes of operation based on audio data captured by at least an audio sensor unit, being part of the aid device 1. Alternatively, the visually impaired or blind person can choose the mode of operation according to his or her needs.

While embodiments of the invention have been illustrated and described, it is not intended that these embodiments illustrate and describe all possible forms of the invention. The words used in the specification are words of description rather than limitation, and it is understood that various changes may be made without departing from the spirit and scope of the invention.

### List of reference numerals

- 1: Aid device (eyeglasses, headband)
- 2: vision sensor unit (event-based sensor / dynamic vision sensor)
- 3: processing unit
- 4: audio output unit / stereo output unit (ear headphone, bone-conducting headphone)
- 5: housing (frame)
- 6: lenses

- S1 to S5, S5', S6 to S11: steps of method
- D1 and D2: questions
- M1 to M3: modes of operation

## Claims

1. A method for transforming visual data into acoustic signals for aiding visually impaired or blind persons, comprising the steps of:
- capturing visual environment data by at least one vision sensor unit (2), wherein the at least one vision sensor unit (2) is formed as an event-based vision sensor,
- transforming the captured visual environment data to acoustic signals, and
- outputting the acoustic signals to the visually impaired or blind person by at least an audio output unit (4).

2. A method according to claim 1, comprising the steps of:
- stereoscopically capturing visual environment data by at least two vision sensor units (2), wherein the at least two vision sensor units (2) are formed as event-based vision sensors,
- extracting 3D visual information from the captured visual environment data,
- transforming the 3D visual information into a 3D stereo sound landscape, and
- outputting the 3D stereo sound landscape to the visually impaired or blind person by at least a stereo output unit (4).

3. The method according to claim 2, **characterized in that** only a part of the extracted 3D visual information is selected to be transformed into the 3D stereo sound landscape.

4. The method according to any of the preceding claims, **characterized in that** a first mode of operation, in which the output acoustic signals are minimized, or a second mode of operation, in which the output acoustic signals are maximized, are carried out.

5. The method according to claim 2 or 3 , **characterized in that** an amount of the extracted 3D visual information to be transformed is selected either manually by the visually impaired or blind person through a user interface or automatically upon detection of the environment by the at least one visual sensor unit and/or at least one audio sensor unit.

6. The method according to any of the preceding claims, **characterized by** the steps of velocity extraction and/or shape detection from and/or clustering of the extracted 3D visual information and the like.

7. The method according to any of the preceding claims, **characterized in that** noise is removed from the captured visual environment data and/or the extracted 3D visual information.

8. The method according to any of the preceding claims, **characterized in that** the 3D stereo sound landscape is encoded by using a head-related transfer function, and/or with added reverberations, and/or a binaural room response.

9. The method according to any of the preceding claims, **characterized in that** characteristic sound representations for known objects are incorporated into the 3D stereo sound landscape.

10. The method according to any of the preceding claims, **characterized in that** the steps of capturing the visual environment data and transforming the captured visual environment data into acoustic signals are carried out in-real time.

11. An aid device, more particularly a travel electronic aid in the form of eye glasses, for visually impaired or blind persons, comprising means for carrying out the method of claims 1 to 10.

12. The aid device according to claim 11, comprising at least one vision sensor unit (2) formed as event-based vision sensor, more particularly dynamic vision sensor.

13. The aid device according to claim 12, further comprising an audio input unit, which is configured to be used for voice control of the device.

14. The aid device according to any of claims 11 to 13, further comprising an audio output unit (4) that has at least an ear headphone and/or at least a bone-conducting headphone.

15. The aid device according to any of claims 11 to 14, further comprising a housing (5), wherein all components of the device are integrated in the housing (5).

16. The aid device according to any of claims 11 to 15, **characterized by** three or more vision sensor units (2) that are arranged in such a way that at least one set of three vision sensor units (2) defines a plane.
